# EUROPEAN PATENT SPECIFICATION

(11) **EP 1 330 989 B1**
(45) Date of publication and mention of the grant of the patent: **07.12.2005**
(21) Application number: 03008510.4
(22) Date of filing: 09.01.1997
(51) Int. Cl.: A61B 18/14

(54) **An electrosurgical instrument**
Elektrochirurgische Vorrichtung
Appareil d'électrochirurgie

(30) Priority: 09.01.1996 GB 9600377; 20.06.1996 GB 9612996
(43) Date of publication of application: 30.07.2003
(62) Divisional of application: 97900315.9
(73) Proprietor: GYRUS MEDICAL LIMITED, Cardiff CF3 0LT (GB)
(72) Inventor: Goble, Colin Charles Owen; Park Close Lodge, Egham, Surrey, TW20 OXE (GB); Goble, Nigel Mark, East Court Farmhouse, Berkshire, RG17 ORH (GB)
(74) Representative: Pratt, David Martin

(56) References cited:
- US-A- 4 976 711
- US-A- 5 431 649
- US-A- 5 437 662
- US-A- 5 507 743

## Description

This invention relates to an electrosurgical instrument for the treatment of tissue in the presence of an electrically-conductive fluid medium, to electrosurgical apparatus including such an instrument, and to an electrode unit for use in such an instrument.

Endoscopic electrosurgery is useful for treating tissue in cavities of the body, and is normally performed in the presence of a distension medium. When the distension medium is a liquid, this is commonly referred to as underwater electrosurgery, this term denoting electrosurgery in which living tissue is treated using an electrosurgical instrument with a treatment electrode or electrodes immersed in liquid at the operation site. A gaseous medium is commonly employed when endoscopic surgery is performed in a distensible body cavity of larger potential volume in which a liquid medium would be unsuitable, as is often the case in laparoscopic or gastroenterological surgery.

Underwater surgery is commonly performed using endoscopic techniques, in which the endoscope itself may provide a conduit (commonly referred to as a working channel) for the passage of an electrode. Alternatively, the endoscope may be specifically adapted (as in a resectoscope) to include means for mounting an electrode, or the electrode may be introduced into a body cavity via a separate access means at an angle with respect to the endoscope - a technique commonly referred to as triangulation. These variations in technique can be subdivided by surgical speciality, where one or other of the techniques has particular advantages given the access route to the specific body cavity. Endoscopes with integral working channels, or those characterised as resectoscopes, are generally employed when the body cavity may be accessed through a natural opening - such as the cervical canal to access the endometrial cavity of the uterus, or the urethra to access the prostate gland and the bladder. Endoscopes specifically designed for use in the endometrial cavity are referred to as hysteroscopes, and those designed for use in the urinary tract include cystoscopes, urethroscopes and resectoscopes. The procedures of transurethal resection or vaporisation of the prostate gland are known as TURP and EVAP respectively. When there is no natural body opening through which an endoscope may be passed, the technique of triangulation is commonly employed. Triangulation is commonly used during underwater endoscopic surgery on joint cavities such as the knee and the shoulder. The endoscope used in these procedures is commonly referred to as an arthroscope.

Electrosurgery is usually carried out using either a monopolar instrument or a bipolar instrument. With monopolar electrosurgery, an active electrode is used in the operating region, and a conductive return plate is secured to the patient's skin. With this arrangement, current passes from the active electrode through the patient's tissues to the external return plate. Since the patient represents a significant portion of the circuit, input power levels have to be high (typically 150 to 250 watts), to compensate for the resistive current limiting of the patient's tissues and, in the case of underwater electrosurgery, power losses due to the fluid medium which is rendered partially conductive by the presence of blood or other body fluids. Using high power with a monopolar arrangement is also hazardous, due to the tissue heating that occurs at the return plate, which can cause severe skin burns. There is also the risk of capacitive coupling between the instrument and patient tissues at the entry point into the body cavity.

With bipolar electrosurgery, a pair of electrodes (an active electrode and a return electrode) are used together at the tissue application site. This arrangement has advantages from the safety standpoint, due to the relative proximity of the two electrodes so that radio frequency currents are limited to the region between the electrodes. However, the depth of effect is directly related to the distance between the two electrodes; and, in applications requiring very small electrodes, the inter-electrode spacing becomes very small, thereby limiting tissue effect and output power. Spacing the electrodes further apart would often obscure vision of the application site, and would require a modification in surgical technique to ensure correct contact of both electrodes with tissue.

There are a number of variations to the basic design of the bipolar probe. For example, U.S. Patent Specification No. 4706667 describes one of the fundamentals of the design, namely that the ratio of the contact areas of the return electrode and of the active electrode is greater than 7:1 and smaller than 20:1 for cutting purposes. This range relates only to cutting electrode configurations. When a bipolar instrument is used for desiccation or coagulation, the ratio of the contact areas of the two electrodes may be reduced to approximately 1:1 to avoid differential electrical stresses occurring at the contact between the tissue and the electrodes.

The electrical junction between the return electrode and tissue can be supported by wetting of the tissue by a conductive solution such as normal saline. This ensures that the surgical effect is limited to the needle or active electrode, with the electric circuit between the two electrodes being completed by the tissue. One of the obvious limitations with the design is that the needle must be completely buried in the tissue to enable the return electrode to complete the circuit. Another problem is one of the orientation: even a relatively small change in application angle from the ideal perpendicular contact with respect to the tissue surface, will change the contact area ratio, so that a surgical effect can occur in the tissue in contact with the return electrode.

Cavity distension provides space for gaining access to the operation site, to improve visualisation, and to allow for manipulation of instruments. In low volume body cavities, particularly where it is desirable to distend the cavity under higher pressure, liquid rather than gas is more commonly used due to better optical characteristics, and because it washes blood away from the operative site.

Conventional underwater electrosurgery has been performed using a non-conductive liquid (such as 1.5% glycine) as an irrigant, or as a distension medium to eliminate electrical conduction losses. Glycine is used in isotonic concentrations to prevent osmotic changes in the blood when intra-vascular absorption occurs. In the course of an operation, veins may be severed, with resultant infusion of the liquid into the circulation, which could cause, among other things, a dilution of serum sodium which can lead to a condition known as water intoxication.

The applicants have found that it is possible to use a conductive liquid medium, such as normal saline, in underwater endoscopic electrosurgery in place of non-conductive, electrolyte-free solutions. Normal saline is the preferred distension medium in underwater endoscopic surgery when electrosurgery is not contemplated, or a non-electrical tissue effect such as laser treatment is being used. Although normal saline (0.9%w/v; 150mmol/l) has an electrical conductivity somewhat greater than that of most body tissue, it has the advantage that displacement by absorption or extravasation from the operative site produces little physiological effect, and the so-called water intoxication effects of non- conductive, electrolyte-free solutions are avoided.

Carbon dioxide is the preferred gaseous distension medium, primarily because of its non- toxic nature and high water solubility.

In endoscopic procedures in which the distension medium is a gas, the applicants have found that it is possible to use an electrically-conductive gas (such as argon) in place of carbon dioxide. Argon is conductive when excited into a discharge state, and has been employed in both endoscopic and conventional monopolar electrosurgery as a method of increasing the distance between the tissue and the instrument, by providing a conductive path between the two when high voltage electrosurgical outputs such as spray or fulgurate are being used. The high voltages used in this application result in a very low penetration of the electrosurgical effect into the tissue, making the technique only suitable to control bleeding from multiple small blood vessels. This allows the surgeon to stanch bleeding from multiple sites in a surgical wound using a rapid "painting" technique, rather than applying electrosurgery to each individual bleeding site. The argon gas is delivered through a hollow surgical instrument, and passes over the monopolar electrode exposed at the tip of the instrument as a stream. This produces a region at the operative site which is rich in argon, and which contributes to the distension of the body cavity. High voltage monopolar electrosurgical outputs are undesirable in endoscopic surgery, because of the risks of damaging structures outside the field of vision, by either capacitive or direct coupling to a portion of the instrument remote from the operative site often outside the field of vision of the operator.

The applicants have developed a bipolar instrument suitable for underwater electrosurgery using a conductive liquid or gaseous medium. This electrosurgical instrument for the treatment of tissue in the presence of a fluid medium, comprises an instrument body having a handpiece and an instrument shaft and an electrode assembly, at one end of the shaft. The electrode assembly comprises a tissue treatment electrode which is exposed at the extreme distal end of the instrument, and a return electrode which is electrically insulated from the tissue treatment electrode and has a fluid contact surface spaced proximally from the exposed part of the tissue treatment electrode. In use of the instrument, the tissue treatment electrode is applied to the tissue to be treated whilst the return electrode, being spaced proximally from the exposed part of the tissue treatment electrode, is normally spaced from the tissue and serves to complete an electrosurgical current loop from the tissue treatment electrode through the tissue and the fluid medium.

The electrode structure of this instrument, in combination with an electrically conductive fluid medium largely avoids the problems experienced with monopolar or bipolar electrosurgery. In particular, input power levels are much lower than those generally necessary with a monopolar arrangement (typically 100 watts). Moreover, because of the relatively large spacing between its electrodes, an improved depth of effect is obtained compared with conventional bipolar arrangement.

US-A-5 507 743 describes a known RF electrode treatment instrument having the features defined in the preamble of claim 1.

The aim of the invention is to provide an improved electrosurgical instrument of this type.

The present invention provides an electrosurgical instrument for the treatment of tissue in the presence of an electrically-conductive fluid medium, the instrument comprising an instrument shaft, and an electrode assembly at one end of the shaft, the electrode assembly comprising a tissue treatment electrode and a return electrode which is electrically insulated from the tissue treatment electrode by means of an insulation member, the tissue treatment electrode being exposed at the distal end portion of the instrument, the exposed end of the tissue treatment electrode being constituted by a single coiled filament, and the return electrode having a fluid contact surface, spaced proximally from the exposed end of the tissue treatment electrode by the insulation member, the coils of the single coiled filament extending laterally through a cut-out (96a) formed in a side surface of the insulation member (96) adjacent to the distal end thereof.

The return electrode is spaced from the tissue treatment electrode so that, in use, it does not contact the tissue to be treated, and so that the electrical circuit is always completed by the conductive fluid, and not simply by arcing between the electrodes. Indeed, the arrangement is such that arcing between the adjacent parts of the electrode assembly is avoided, thereby ensuring that the tissue treatment electrode can become enveloped in a vapour pocket so that tissue entering the vapour pocket becomes the preferred path for current to flow back to the return electrode via the conductive fluid.

The electrosurgical instrument of the invention is useful for dissection, resection, vaporisation, desiccation and coagulation of tissue and combinations of these functions with particular application in hysteroscopic surgical procedures. Hysteroscopic operative procedures may include: removal of submucosal fibroids, polyps and malignant neoplasms; resection of congenital uterine anomalys such as septum or subseptum; division of synechiae (adhesiolysis); ablation of diseased or hypertrophic endometrial tissue; and haemostasis.

The instrument of the invention is also useful for dissection, resection, vaporisation, desiccation and coagulation of tissue and combinations of these functions with particular application in arthroscopic surgery as it pertains to endoscopic and percutaneous procedures performed on joints of the body including, but not limited to, such techniques as they apply to the spine and other non-synovial joints. Arthroscopic anterior release of the temperomandibular joint; synovectomy, cartilage debridement, chondroplasty, division of intra-articular adhesions, fracture and tendon debridement as applied to any of the synovial joints of the body; inducing thermal shrinkage of joint capsules as a treatment for recurrent dislocation, subluxation or repetitive stress injury to any articulated joint of the body; disectomy either in the treatment of disc prolpase or as part of a spinal fusion via a posterior or anterior approach to the cervical, thoracic and lumbar spine or any other fibrous joint for similar purposes; excision of diseased tissue; and haemostasis.

The instrument of the invention is also useful for dissection, resection, vaporisation, desiccation and coagulation of tissue and combinations of these functions with particular application in urological endoscopic (urethroscopy, cystoscopy, ureteroscopy and nephroscopy) and percutaneous surgery. Urological procedures may include: electro- vaporisation of the prostate gland (EVAP) and other variants of the procedure commonly referred to as transurethral resection of the prostate (TURP) including, but not limited to, interstitial ablation of the prostate gland by a percutaneous or perurethral route whether performed for benign or malignant disease; transurethral or percutaneous resection of urinary tract tumours as they may arise as primary or secondary neoplasms and further as they may arise anywhere in the urological tract from the calyces of the kidney to the external urethral meatus; division of strictures as they may arise at the pelviureteric junction (PUJ), ureter, ureteral orifice, bladder neck or urethra; correction of ureterocoele; shrinkage of bladder diverticular; cystoplasty procedures as they pertain to corrections of voiding dysfunction; thermally induced shrinkage of pelvic floor as a corrective treatment for bladder neck descent; excision of diseased tissue; and haemostasis.

Surgical procedures using the instrument of the invention include introducing the electrode assembly to the surgical site through an artificial conduit (a cannula), or through a natural conduit which may be in an anatomical body cavity or space or one created surgically. The cavity or space may be distended during the procedure using a fluid or may be naturally held open by anatomical structures. The surgical site may be bathed in a continuous flow of conductive fluid such as saline solution to fill and distend the cavity. The procedures may include simultaneous viewing of the site via an endoscope or using an indirect visualisation means.

Conveniently, the return electrode is formed with a hood-like extension which extends over the surface of the insulation member which is opposite the cut-out.

Advantageously, the single coiled filament is connected to a central conductor, the insulation member surrounding the central conductor.

The single coiled filament may be made from a precious metal such as platinum or from a platinum alloy such as platinum/iridium, platinum/tungsten or platinum/cobalt. The single coiled filament could also be made of tungsten. The insulation member may be made of a ceramic material, silicone rubber or glass.

The invention further provides electrosurgical apparatus comprising a radio frequency generator and an electrosurgical instrument as defined above.

Advantageously, the radio frequency generator includes control means for varying the output power delivered to the electrodes, the control means being such as to provide output power in first and second output ranges, the first output range being for powering the electrosurgical instrument for tissue dessication, and the second output range being for powering the electrosurgical instrument for tissue removal by vaporisation. Preferably, the first output range is from about 150 volts to 200 volts, and the second output range is from about 250 volts to 600 volts, the voltage being peak voltages.

The invention will now be described in greater detail, by way of example with reference to Fig 11c. The electrode units shown in Figs. 1 to 10, 11a, 11b and 11d do not form part of the present invention.
Figure 1 is a diagrammatic side elevation of an electrode assembly at a distal end of a first form of electrode unit which is not part of the present invention;
Figure 2 is a graph illustrating the hysteresis which exists between the use of the electrode unit of Figure 1 in desiccating and vaporising modes;
Figure 3a is a diagrammatic side elevation of the first electrode unit, showing the use of such a unit for tissue removal by vaporisation;
Figure 3b is a diagrammatic side elevation of the first electrode unit, showing the use of such a unit for tissue desiccation;
Figures 4a to 4c are diagrammatic side elevations of the electrode assembly of a second form of electrode unit which is not part of the present invention;
Figures 5a and 5b are diagrammatic side elevations of the electrode assembly of a third form of electrode unit which is not part of the present invention;
Figures 6a and 6b are diagrammatic side elevations of the electrode assembly of a fourth form of electrode unit which is not part of the present invention;
Figures 7a and 7b are diagrammatic side elevations of a fifth form of electrode unit which is not part of the present invention; Figure 8 is a diagrammatic side elevation of a sixth form of electrode unit which is not part of the present invention;
Figure 9 is a cross-section taken on the line A-A of Figure 8;
Figure 10 is a diagrammatic side elevation of a seventh form of electrode unit which is not part of the present invention;
Figures 11a, 11b and 11d are diagrammatic side elevations of forms of electrode unit which is not part of the present invention;
Figure 11c is a diagrammtic side elevation of an electrode unit constructed in accordance with the invention; and
Figure 12 is a diagram showing an electrosurgical apparatus constructed in accordance with the invention.

Each of the electrode units described below is intended to be used with a conductive distension medium such as normal saline, and each unit has a dual-electrode structure, with the conductive medium acting as a conductor between the tissue being treated and one of the electrodes, hereinafter called the return electrode. The other electrode is applied directly to the tissue, and is hereinafter called the tissue treatment (active) electrode. In many cases, the use of a liquid distension medium is preferable, as it prevents excessive electrode temperatures in most circumstances, and largely eliminates tissue sticking.

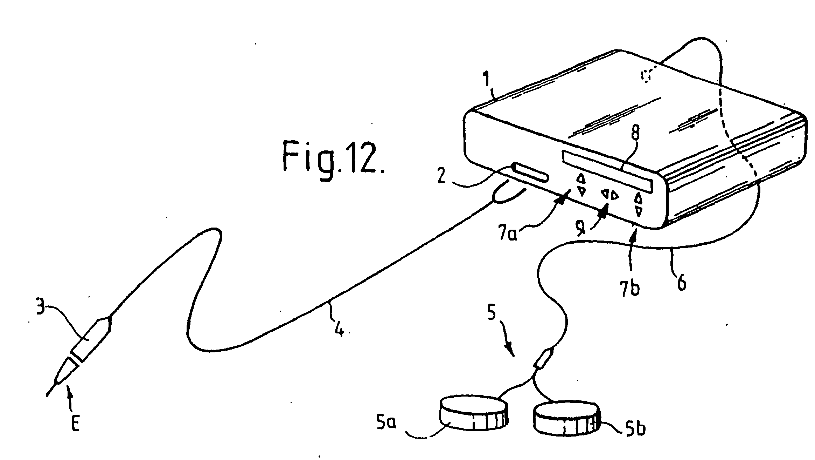

Referring to the drawings, Figure 12 shows electrosurgical apparatus including a generator 1 having an output socket 2 providing a radio frequency (RF) output for an instrument in the form of a handpiece 3 via a connection cord 4. Activation of the generator 1 may be performed from the handpiece 3 via a control connection in the cord 4, or by means of a footswitch unit 5, as shown, connected separately to the rear of the generator 1 by a footswitch connection cord 6. In the illustrated embodiment, the footswitch unit 5 has two footswitches 5a and 5b for selecting a desiccation mode and a vaporisation mode of the generator 1 respectively. The generator front panel has push buttons 7a and 7b for respectively setting desiccation and vaporisation power levels, which are indicated in a display 8. Push buttons 9a are provided as an alternative means for selection between the desiccation and vaporisation modes.

The handpiece 3 mounts a detachable electrode unit E, such as the electrode units E1 to E 11 to be described below.

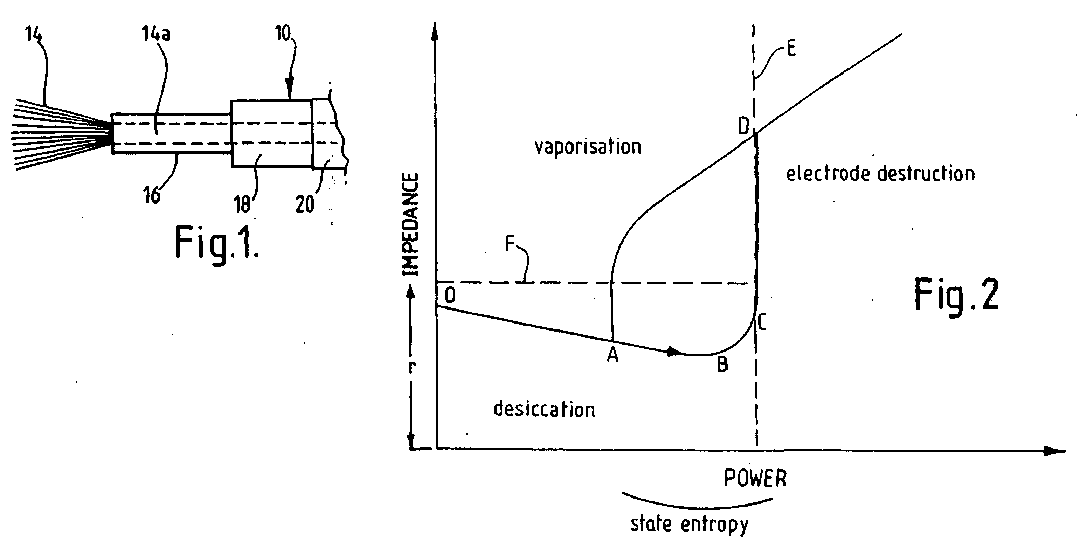

Figure 1 shows the first form of electrode unit E1 for detachable fastening to the electrosurgical instrument handpiece 3, the electrode unit comprising a shaft 10, which is constituted by a semi-flexible tube made of stainless steel or phynox electroplated in copper or gold, with an electrode assembly 12 at a distal end thereof. At the other end (not shown) of the shaft 10, means are provided for connecting the electrode unit E1 to a handpiece both mechanically and electrically.

The RF generator 1 (not shown in Figure 1) delivers an electro-surgical current to the electrode assembly 12. The generator includes means for varying the delivered output power to suit different electrosurgical requirements. The generator may be as described in the specification of our European Patent 0754437.

The electrode assembly 12 includes a central, tissue treatment (active) electrode 14 in the form of a brush electrode. The active electrode 14 is connected to the generator 1 via an integral central conductor 14a and a central copper conductor (not shown) positioned within the handpiece of the instrument. The brush electrode 14 is constituted by a plurality of filaments of tungsten, the filaments having diameters lying in the range from 0.05mm to 0.3mm. A tapered ceramic insulation sleeve 16 surrounds the conductor 14a. A return electrode 18, which is constituted by the distal end portion of the shaft 10, abuts the proximal end of the sleeve 16. An outer insulating coating 20 surrounds the proximal portion of the shaft adjacent to the return electrode 18. The coating 20 would be polyvinylidene fluoride, a polyimide, polytetrafuoroethylene, a polyolefin, a polyester or ethylene tetrafluoroethylene.

By varying the output of the generator 1, the electrode unit E1 of Figure 1 can be used for tissue removal by vaporisation, or for desiccation. Figure 2 illustrates how the RF generator 1 can be controlled to take advantage of the hysteresis which exists between the desiccation and the vaporising modes of the electrode unit E1. Thus, assuming the electrode assembly 12 of the unit E1 is immersed in a conductive medium such as saline, there is an initial impedance "r" at point "O", the magnitude of which is defined by the geometry of the electrode assembly and the electrical conductivity of the fluid medium. The value of "r" will change when the active electrode 14 contacts tissue, the higher the value of "r" the greater the propensity of the electrode assembly 12 to enter the vaporisation mode. When RF power is applied to the electrode assembly 12 the fluid medium heats up. Assuming the fluid medium is normal saline (0.9% w/v), the temperature coefficient of the fluid medium is positive, so that the corresponding impedance coefficient is negative. Thus, as power is applied, the impedance initially falls and continues to fall with increasing power to point "B", at which point the saline in intimate contact with the electrode assembly 12 reaches boiling point. Small vapour bubbles form on the surface of the active electrode 14 and the impedance then starts to rise. After point "B", as power is increased further, the positive power coefficient of impedance is dominant, so that increasing power now brings about increasing impedance.

As a vapour pocket forms from the vapour bubbles, there is an increase in the power density at the residual electrode/saline interface. There is, however, an exposed area of the active electrode 14 not covered by vapour bubbles, and this further stresses the interface, producing more vapour bubbles and thus even higher power density. This is a run-away condition, with an equilibrium point only occurring once the electrode is completely enveloped in vapour. For given set of variables, there is a power threshold before this new equilibrium can be reached (point "C").

The region of the graph between the points "B" and "C", therefore, represents the upper limit of the desiccation mode. Once in the vaporisation equilibrium state, the impedance rapidly increases to around 1000 ohms, with the absolute value depending on the system variables. The vapour pocket is then sustained by discharges across the vapour pocket between the active electrode 14 and the vapour/saline interface. The majority of power dissipation occurs within this pocket, with consequent heating of the active electrode 14. The amount of energy dissipation, and the size of the pocket, depends on the output voltage. If this is too low, the pocket will not be sustained, and if it is too high the electrode assembly 12 will be destroyed. Thus, in order to prevent destruction of the electrode assembly 12, the power output of the generator 1 must be reduced once the impedance has reached the point "D". It should be noted that, if the power is not reduced at this point, the power/impedance curve will continue to climb and electrode destruction would occur. The dotted line E indicates the power level above which electrode destruction is inevitable. As the power is reduced, the impedance falls until, at point "A", the vapour pocket collapses and the electrode assembly 12 reverts to the desiccation mode. At this point, power dissipation within the vapour pocket is insufficient to sustain it, so that direct contact between the active electrode 14 and the saline is re-established, and the impedance falls dramatically. The power density at the active electrode 14 also falls, so that the temperature of the saline falls below boiling point. The electrode assembly 12 is then in a stable desiccation mode. With the generator described in the specification of our European Patent 0754437, the output is 350 to 550 volts peak for the vaporisation mode, and about 170 volts peak for the desiccation mode.

It will be apparent that the electrode unit E1 of Figure 1 can be used for desiccation by operating the unit in the region of the graph between the point "0" and a point in the region between the points "B" and "C". In this case, the electrode assembly 12 would be introduced into a selected operation site with the active electrode 14 adjacent to the tissue to be treated, and with the tissue, the active electrode and the return electrode 18 immersed in the saline. The RF generator 1 would then be activated (and cyclically controlled as described in the specification of our European Patent 0754437) to supply sufficient power to the electrode assembly 12 to maintain the saline adjacent to the active electrode 14 at, or just below, its boiling point without creating a vapour pocket surrounding the active tip. The electrode assembly would then be manipulated to cause heating and desiccation of the tissue in a required region adjacent to the active electrode 14. The electrode unit E1 can be used for vaporisation in the region of the graph between the point "D" and the dotted line F which constitutes the level below which vaporisation cannot occur. The upper part of this curve is used for tissue removal by vaporisation. It should also be appreciated that the electrode unit E1 could be used for cutting tissue. In the cutting mode, the electrode unit E1 still operates with a vapour pocket, but this pocket is much smaller than that used for vaporisation, so that there is the least amount of tissue damage commensurate with cutting. Typically, the generator operates at about 270 volts peak for cutting.

The temperature generated at the active electrode 14 is of the order of 1500°C in the vaporisation mode, so that the active electrode is made of a material that can withstand such high temperatures. Preferably, the active electrode 14 is made of tungsten, platinum or a platinum alloy (such as platinum/iridium or platinum/tungsten).

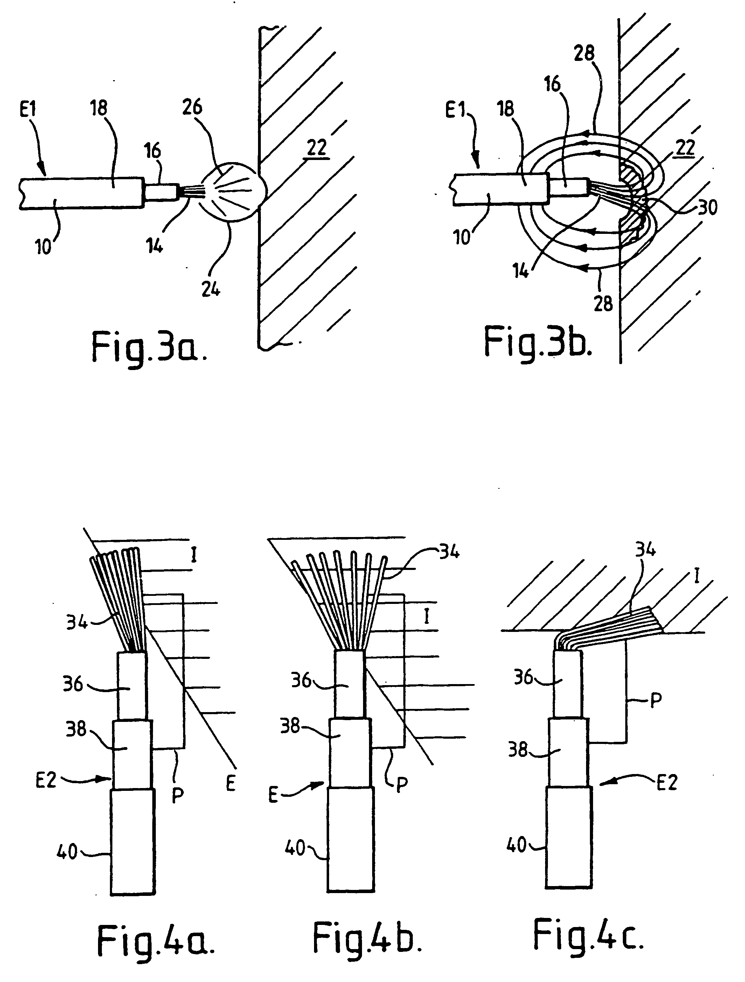

Figure 3a illustrates schematically the use of the electrode unit E1 of Figure 1 for tissue removal by vaporisation. Thus, the electrode unit E1 creates a sufficiently high energy density at the active electrode 14 to vaporise tissue 22, and to create a vapour pocket 24 surrounding the active electrode. The formation of the vapour pocket 24 creates about a 10-fold increase in contact impedance, with a consequent increase in output voltage. Arcs 26 are created in the vapour pocket 24 to complete the circuit to the return electrode 18. Tissue 22 which contacts the vapour pocket 24 will represent a path of least electrical resistance to complete the circuit. The closer the tissue 22 comes to the active electrode 14, the more energy is concentrated to the tissue, to the extent that the cells explode as they are struck by the arcs 26, because the return path through the conductive fluid (saline in this case) is blocked by the high impedance barrier of the vapour pocket 24. The saline solution also acts to dissolve the solid products of vaporisation.

Figure 3b illustrates schematically the use of the electrode unit E1 for tissue desiccation. In the desiccation mode, output power is delivered to the electrode assembly 12 in a first output range, so that current flows from the active electrode 14 to become heated, preferably to a point at or near the boiling point of the saline solution. This creates small vapour bubbles on the surface of the active electrode 14 that increases the impedance about the active electrode.

The body tissue 22 typically has a lower impedance than the impedance of the combination of vapour bubbles and saline solution adjacent to the active electrode 14. When the active electrode 14 surrounded by small vapour bubbles and saline solution is brought into contact with the tissue 22, the tissue becomes part of the preferred electrical current path. Accordingly, the preferred current path goes out of the active electrode 14 at the point of tissue contact, through the tissue 22, and then back to the return electrode 18 via the saline solution, as shown by the current path lines 28 in Figure 3b.

The electrode unit E1 has particular application in desiccating tissue. For tissue desiccating, one preferred approach is to contact only part of the active electrode 14 to the tissue 22, with the remainder of the active electrode remaining remote from the tissue and surrounded by saline solution, so that current can pass from the active electrode to the return electrode 18 via the saline solution, without passing through the tissue. For example, in the embodiment shown in Figure 3b, only the distal portion of the active electrode 14 contacts the tissue 22, with the proximal portion remaining spaced away from the tissue.

The electrode unit E1 can achieve desiccation with no or minimal charring of the tissue 22. When the active electrode 14 contacts the tissue 22, current passes through the tissue, causing the tissue at, and around, the contact point to desiccate. The area and volume of desiccated tissue 30 expands generally radially outwardly from the point of contact. As the tissue 22 is desiccated, it loses its conductivity. As the area and volume of desiccated tissue 30 grows, a point is reached where the conductivity of the tissue is less than the conductivity of the heated saline solution surrounding the active electrode 14.

The current will prefer to follow the least-impedance path. Accordingly, as the impedance of the tissue 22 increases (due to desiccation) to a point where it approaches or exceeds the impedance of the combination of vapour bubbles and saline solution surrounding the active electrode 14, the preferred electrical current path will shift to a new path through the vapour bubbles and saline solution. Accordingly, once a large enough portion of tissue is desiccated, most (or substantially all) the current flow necessarily shifts to pass directly from the active electrode 14 into the saline solution. Before the tissue 22 becomes charred or scorched, the increased impedance of the desiccated tissue 30 causes most of the current to follow the path through the saline solution. No current, or a very small amount of current, will continue to pass through the desiccated tissue, and charring will be prevented.

In the embodiment shown in Figure 3b, the exposed, stranded portion of the active electrode 14 allows parts of the active electrode to contact the tissue surface, while still maintaining most of the active electrode exposed portion out of contact with the tissue. Because most of the exposed portion of the active electrode 14 is out of contact with the tissue 22, the current path will more easily shift, upon desiccation of a sufficient tissue volume, from the path through the tissue to a path that goes directly from the active electrode to the saline solution.

When the electrode unit E1 is in the desiccation mode, the flexibility of the brush electrode 14 offers considerable advantages when working with small diameter electrodes in irregular body cavities in which large areas of tissue require desiccation. From a technical standpoint, the return:active ratio is variable from > 1:1 in the "closed" form to < 1:1 in the "splayed" form. This variability of the return:active ratio is explained in greater detail below with reference to Figures 4a to 4c.

Figure 4 shows the second form of electrode unit E2 whose electrode assembly 32 includes an active electrode 34 which is constituted by a plurality of filaments made of a conductive material such as stainless steel. The filaments of the brush electrode 34 are much longer (10mm as compared with 5mm) than the filaments of the brush electrode 14, as the electrode unit E2 is intended primarily for desiccation. In this embodiment, the return:active ratio is variable from > 2:1 in the "closed" form to < 1:1 in the "splayed" form. The electrode assembly 32 also includes a ceramic insulation sleeve 36, a return electrode 38 and an outer insulating sheath 40. The active electrode 34 is a brush electrode whose tip is flexible to provide a reproducible tissue effect which is substantially independent of the application angle of the electrode with respect to the surface of the tissue T (see Figure 4c). Thus, the flexibility of the active electrode 34 results in differential contact areas of the active electrode dependent on the applied pressure. For example, Figure 4a shows the brush electrode 34 "closed" during the application of light pressure, and Figure 4b shows the brush "splayed" by firm tissue pressure. This enables the creation of a broader surgical effect than the diameter of the electrode 34 would otherwise allow, thereby reducing treatment time. Figures 4a to 4c also show the return path P for the current flow from the active electrode 34 to the return electrode 38 via the conductive medium.

This large variation in the return:active ratio is a feature which cannot be supported by conventional bipolar designs. This variation in ratio can occur because the conductive path to complete the electrical circuit is maintained by the low impedance of the electrode contact with the conductive fluid operating medium. In order to sustain the low impedance transfer of RF energy to the tissues, the RF generator must be controlled in such a way that vapour pockets cannot form at the interface between the active electrode and the tissue. This allows the tissue contact to be continually wetted by the conductive fluid so that, whilst the tissue water is removed by thermal desiccation, the impedance reaches an upper limit determined by a point just below a voltage threshold above which vapour pockets will start to form. This, combined with the greater insulation separation between the active and return electrodes, enables this type of electrode unit to deliver much higher powers effectively to the tissue for a given electrode dimension than any known electrode unit.

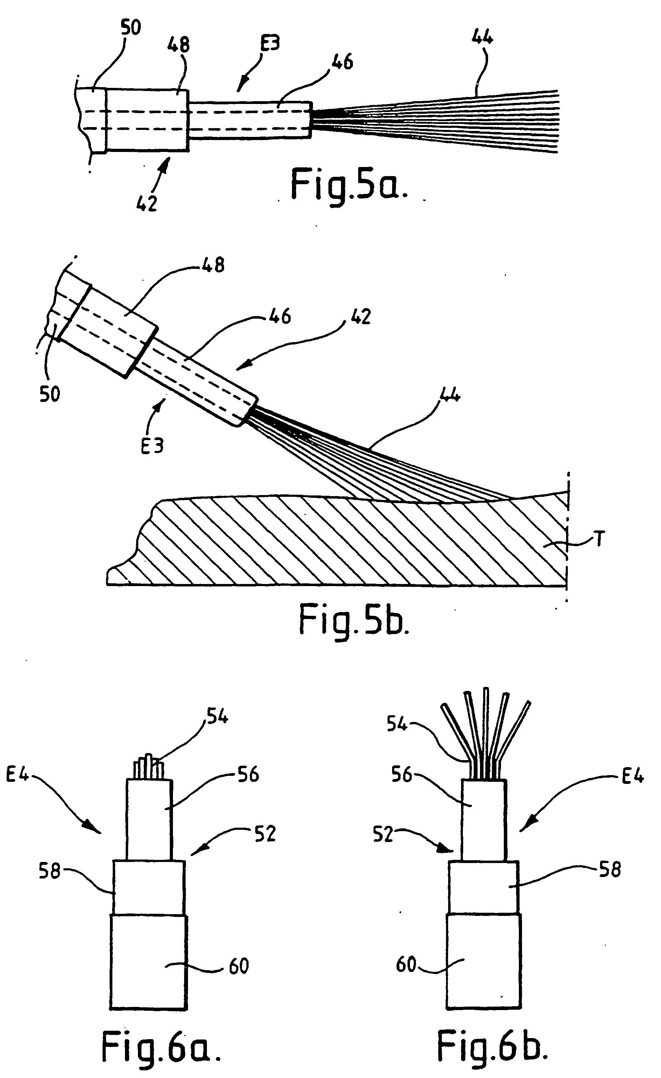

Figures 5a and 5b show the third form of electrode unit E3. This unit E3 is a modification of the electrode unit E2, and its electrode assembly 42 includes an active electrode 44 which is constituted by plurality of filaments made of stainless steel. The active electrode 44 is, therefore, a brush electrode and the filaments of this electrode are of a similar length to the filaments of the brush electrode 32. The electrode unit E3 is, therefore, intended primarily for desiccation. The electrode assembly 42 also includes a ceramic insulation sleeve 46, a return electrode 48 and an outer insulating sheath 50. The insulation sleeve 46 is made of a ceramic material and, like the insulation sleeve 16 of the electrode unit E1, it tapers towards the distal end of the electrode assembly 42.

Figure 5a shows the electrode unit E3 in a non-operational position, and Figure 5b shows the unit in desiccating mode against tissue T.

Figures 6a and 6b show a fourth form of electrode unit E4 whose electrode assembly 52 includes an extensible active electrode 54 in the form of a brush electrode. The filaments of the brush electrode 54 are made of tungsten, platinum, platinum/tungsten or platinum/iridium. The electrode unit E4 also includes a ceramic insulation sleeve 56, a return electrode 58, and an insulating sheath 60. As shown in Figure 6a, the active electrode 54 can be withdrawn substantially within the insulation sleeve 56 so that only the free end portions of its filaments are exposed. With the active electrode 54 in this position, the electrode unit E4 can be used to vaporise tissue in the manner described above with reference to Figure 3. On the other hand, if the active electrode 54 is extended (see Figure 6b), so that its filaments extend fully from the distal end of the sleeve 56, the electrode unit E4 can be used for desiccation. The ratio of the contact areas of the return to active electrodes of the unit E4 can, therefore, be varied between the fully retracted active electrode position (in which the ratio is high and the unit is used for vaporisation), and the extended position (in which the ratio is low and the unit is used for desiccation). The unit E4 achieves its dual functionality by varying the extent by which the filaments of the active electrode 54 are extended. Dual functionality could also be achieved by varying axial separation between the active electrode 54 and the return electrode 58 (for example by varying the length of the insulation sleeve 56). With a large extension of the filaments of the active electrode 54 or with a large axial electrode separation, a large electric field is created, so that more tissue is affected. With no extension of the filaments of the active electrode 54 or with a reduced electrode separation, a smaller electric field is produced, and is used for cutting or vaporisation in circumstances where no collateral thermal damage to tissue is desirable. The larger electric field pattern is desirable for desiccation, or in circumstances where the desiccation of collateral tissue is desirable to prevent haemorrhage from a cut surface.

Depending upon the ratio of the return:active electrode area, therefore, the brush electrode unit E4 can have a desiccation function (as exemplified by the embodiments of Figures 4 and 5), a vaporisation function (as exemplified by the embodiment of Figure 3), or a dual desiccation/vaporisation function (as exemplified by the embodiment of Figure 6).

As indicated above, the primary use for the desiccating brush is in providing a flexible, broad area electrode for desiccating large irregular areas of tissue. The requirement to treat such areas occurs in hysteroscopic surgery - desiccation of the endometrial lining of the uterus, and in urological surgery - desiccation and shrinkage of bladder diverticular. In both instances, the electrode is introduced through the working channel of the endoscope.

Introduction of the desiccating brush with a long and flexible, filamentary structure can prove problematical when the working channel of the endoscope is angled or includes steps in the inner bore. This can deform the brush filaments which, once inserted, cannot be adjusted and may not conform to the area of tissue to be treated. Bending back of the filaments may also inadvertently create an electrical short to the return electrode.

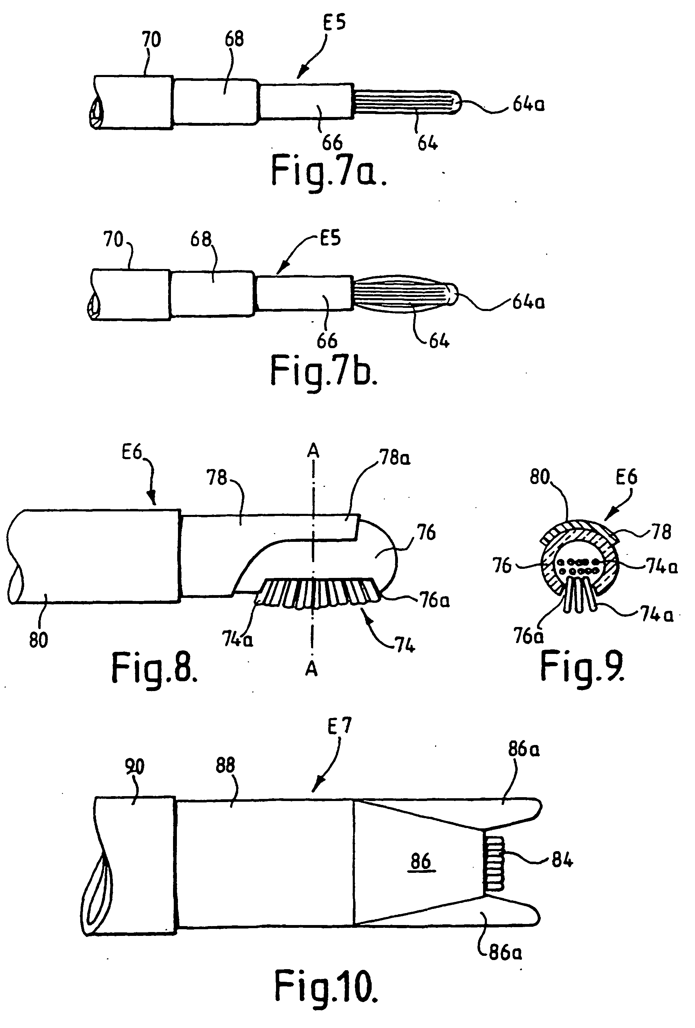

Whilst preserving the desired functions of flexibility and contact area geometry dependent on the pressure of application, the basic desiccating brush can be modified to overcome this problem. For example, the brush filaments can be simply twisted together. Preferably, however, the filaments are welded together at their distal ends as shown in Figure 7 which shows a fifth form of electrode unit E5. The electrode unit E5 includes an active electrode 64 in the form of a brush electrode whose filaments are made of platinum, platinum/tungsten or platinum/iridium. The distal ends 64a of the filaments are welded together as shown in Figure 7a. This prevents distortion of the filaments in the working channel of an endoscope, whilst permitting bowing of the filaments (as shown in Figure 7b) to increase tissue contact area. The electrode unit E5 includes a ceramic insulation sleeve 66, a return electrode 68 and an outer insulating sleeve 70.

In the dual function brush electrode, the return:active electrode area can be elevated to a level which is capable of producing tissue vaporisation. Obviously, with a very small active electrode area at the extreme of this range, the amount of tissue which can be desiccated becomes too small to be practically useful. If, however, the ratio is configured in the mid- range, then the same electrode can be used to produce both effective desiccation and tissue removal by vaporisation. The short brush described in Figure 1 is one example of such a dual purpose electrode. Given that the filaments cannot be fabricated in stainless steel to support vaporisation, tungsten filaments are the preferred material in the short brush due to their rigidity overcoming the issues of distortion during introduction. Platinum alloys withstand the high vaporisation temperatures better than tungsten but, due to their flexibility and the annealing process during use, cannot be used in the short brush form. Platinum alloy dual-function brush-type electrodes, therefore, require the modifications of twisting, braiding, or welding of the distal tips to prevent distortion.

These combined multi-functional brush electrode forms are particularly useful in removing tumour masses or polyps encountered during hysteroscopic and urological surgery. They can vaporise the tumour bulk, incise the stalks of polyps, and desiccate any bleeding vessels or the base of the tumour without the need to change electrodes.

In these multi-functional forms, the active electrode area is maximised for desiccation whilst still being capable of vaporisation or cutting functions. The minimum ratio depends on four important criteria, namely:
1. The intrinsic impedance of the target tissue.
2. The volume of the body cavity.
3. The configuration of the active electrode.
4. The maximum output power from the RF generator.

The configuration of the active electrode obviously influences the ratio, with cylindrical forms representing the lowest ratio for a given length, but the other factors relate to the inability of the electrode to retain a vapour bubble. The filaments of the brush-type electrodes retain vapour bubbles, which helps maintain the vaporisation condition.

An arthroscope electrode may be characterised as short (100-140mm), rigid with a working diameter up to 4mm. It can be introduced through a stab incision into a joint cavity (with or without a cannula) using the triangulation technique. When an arthroscope includes a brush electrode of the type described above, it is operated with a motion which commonly moves the brush electrode between the 9 o'clock and 3 o'clock positions on the arthroscopic image. As a result, the tissue to be treated is commonly approached at a shallow working angle with respect to the axis of the electrode. The electrode for arthoscopy thus needs to have an effect consistent with this angled approach to the tissue. The tissue to be treated, such as meniscal cartilage, is commonly dense and of a high electrical impedance, such tissue having a free edge representing a common injury site where treatment is required. The drawback of known arthroscope electrodes which are solid form electrodes is that, because the joint spaces are commonly small (the joint spaces in the knee being typically 60-100 mls under fluid distension), the vapour bubbles generated are large and tend to cause problems with visualisation.

Figure 8 shows an arthroscope electrode unit E6. The electrode unit E6 includes an active electrode 74 which is constituted by a plurality of filaments made of tungsten or an alloy of tungsten or platinum. The active (brush) electrode 74 is connected to an RF generator (not shown) via a central copper conductor (also not shown). A ceramic insulation sleeve 76 surrounds the central conductor, the filaments 74a of the brush electrode passing along the insulation sleeve and extending laterally therefrom through a cut-out 76a. A return electrode 78, which is constituted by the distal end of the instrument shaft, surrounds the proximal end of the sleeve 76. An outer insulating coating 80 (which would be polyvinylidene fluoride, a polyimide, polytetrafluoroethylene, a polyolefin, a polyester or ethylene tetrafluoroethylene) surrounds the proximal portion of the shaft adjacent to the return electrode 78. The return electrode 78 is formed with a hood-like extension 78a which extends over the surface of the sleeve 76 which is opposite to the cut-out 76a. The electrode unit E6 can, thus, provide maximum tissue engagement for shallow working angle applications, and is known as a side-effect electrode.

Because of the higher impedance of the target tissue, the arthroscopic multi-function brush electrode should support a lower ratio than electrodes designed for hysteroscopic and urological applications where the tissue is more vascular. Reducing the ratio does, however, have one drawback in body cavities of small volume, such as the knee joint which is typically 60-80 mls, and that is heating of the surrounded irrigant or distension fluid. Heating occurs primarily during the application of power to reach the vaporisation threshold. Once the threshold has been reached, the power requirement typically falls by 30-50%. Reducing the electrode ratio increases the power requirement to reach the threshold so that, despite the high impedance of the target tissue, it is undesirable to reduce the ratio to the lowest value capable of supporting vaporisation.

In addition, the high impedance is due to lack of vascularity of such tissues as meniscal cartilage. Except, therefore, when muscle or synovial tissue is being treated, the primary function of the athroscopic brush electrode is that it should provide rapid debulking of dense, avascular tissue. Desiccate functionality is not a requirement of such an instrument. Indeed, very short rigid brush electrodes with electrode ratios greater than 5:1 are desirable. The only reason for not elevating the ratio further is the need to engage the maximum amount of tissue and simultaneously reduce procedure time.

A short, rigid brush electrode (of the type described above with reference to Figure 1 or Figure 6a) can be thought of as an end-effect electrode which has tissue debulking precision with minimal thermal spread. Consequently, it can be used to create discrete holes in tissue, thereby to create an access channel to tissue deep to the surface, as may be required as part of an interstitial ablation technique on a tissue mass such as a prostate adenoma or a uterine fibroid (myolysis). This use of a vaporising, end-effect, technique enables only the fibroid to be removed by complete debulking leaving a resection margin conforming to the "false capsule" of the fibroid. No normal tissue is removed and, due to control of collateral thermal effects at the endometrial resection margin, the scarring is reduced to a minimum, thereby increasing what chances there were of restoring fertility. Additionally, of course, vaporisation does not produce resection chippings to interfere with visualisation and prolong the procedure through the need to wash them out once the resection is completed. Conventional loop electrode resectoscopes require removal of normal tissue surrounding such fibroids, and this is disadvantageous because it increases the chance of bleeding, the risk of uterine perforation and the scarring of the uterus. This latter aspect is particularly undesirable when the procedure is being performed in an attempt to restore fertility.

Alternatively, a short, rigid brush electrode can be used to debulk a tumour (such as a fibroid, a bladder tumour or a prostate adenoma), or it can be used with the multiple puncture or drilling technique. In this case, after removing the intrauterine portion, the intramural portion can be treated by creating ("drilling") a series of holes into the abnormal tissue whether, for example, this is a fibroid or prostatic adenoma. To assess the depth of penetration, marks may be provided on the electrode shaft at measured distances from the tip, and hence to compare the depth of penetration against the pre-operative results of tests performed to establish size of the tumour or adenoma. The residual tissue bridges will shrink as part of the healing process. Whilst not removing the whole tumour, this technique is safer and quicker than removing the entire fibroid or prostatic adenoma, when treatment is being performed either for menorrhagia or bladder outflow obstruction, respectively.

Another problem with working in the confined space of a joint cavity is in preventing damage to adjacent structures, particularly when the vaporising effect is enhanced, and both the tissue density and application angle make engagement and location difficult. This protection feature is intrinsic in the side-effect brush of Figure 8, when the insulation sleeve 76 protects tissue above, below and behind the active electrode window 76a which only occupies a small arc of the cross-sectional form (as shown in Figure 9).

Figure 10 shows the electrode assembly of the seventh form of electrode unit E7. This electrode assembly includes a central, tissue treatment (active) electrode constituted by a plurality of filaments made of tungsten or an alloy of tungsten or platinum, a tapered ceramic insulation sleeve 86, a return electrode 88, and an outer insulating sleeve 90. The insulation sleeve 86 is formed with a pair of diametrically-opposed, forwardly-extending wings 86a which project beyond the active electrode 84. The filaments constituting the active electrode 86 extend only a short distance from the distal end of the insulation sleeve 86, thereby constituting a very short brush electrode. The electrode unit E7 has, therefore, a large return:active electrode ratio, so that this electrode unit is intended primarily for a tissue removal by vaporisation. The electrode unit E7 is particularly useful for electrosurgical operations on meniscal cartilage or any other elongate laminate structure which is to be treated from the side, as the wings 86a can be used to trap the cartilage against the active electrode 84. The configuration of the wings 86a also assists in preventing unnecessary exposure of the active electrode 84, which may otherwise damage adjacent structures when working in the confined spaces commonly encountered in endoscopic surgery.

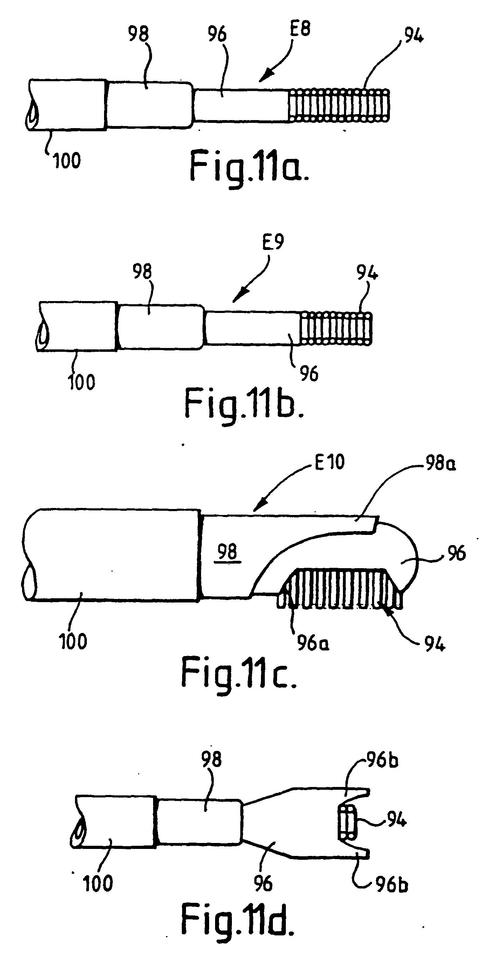

Figures 11a to 11d show forms of electrode unit E8 to E11, each of which incorporates an active electrode in the form of a coiled spring filament 94. The electrode units E8 to E11 each includes an insulation sleeve 96, a return electrode 98 and an insulating sheath 100. The electrode unit E8 of Figure 11a is similar to that of Figure 5a, being intended primarily for desiccation; and the electrode unit E9 of Figure 11b is similar to that of Figure 1, being intended primarily for vaporisation. The electrode unit E10 of Figure 11c, which is constructed in accordance with the invention, is similar to that of Figures 8 and 9, in that the coil electrode 94 is formed in a cut-out 96a formed in the side of the insulation sleeve 96, and the return electrode 98 is formed with a hood-like extension 98a which extends over the surface of the sleeve 96 which is opposite to the cut-out 96a. The electrode unit E10 can, thus, provide maximum tissue engagement for shallow working angle applications, and is another form of side-effect electrode. The electrode unit E11 of Figure 11d is similar to that of Figure 10, in that the insulation sleeve 96 is formed with a pair of diametrically-opposed, forwardly-extending wings 96b. In each of these embodiments, the active electrode 94 is made of an alloy of platinum.

The electrode units E8 to E11 are similar to the brush-type electrodes of Figures 1 to 10, and have similar surgical effects, apart from the fact that they eliminate the risk of splaying (which is advantageous in certain electro-surgical procedures). They have, however, the advantage of simplifying the assembly procedure, particularly when using platinum alloy materials.

It will be apparent that modification could be made to the electrosurgical instruments described above. For example, the insulation sleeves 16, 36, 46, 56, 66, 76, 86 and 96 could be made of a silicone rubber (such as a silicone polyurethene), glass, a polyimide or a thermoplastics material.

## Claims

1. An electrosurgical instrument for the treatment of tissue in the presence of an electrically-conductive fluid medium, the instrument comprising an instrument shaft (10), and an electrode assembly (E10) at one end of the shaft, the electrode assembly comprising a tissue treatment electrode (94) and a return electrode (98) which is electrically insulated from the tissue treatment electrode by means of an insulation member (96), the tissue treatment electrode being exposed at the distal end portion of the instrument, the exposed end of the tissue treatment electrode being constituted by a single coiled filament (94), and the return electrode having a fluid contact surface, spaced proximally from the exposed end of the tissue treatment electrode by the insulation member, **characterised in that** the coils of the single coiled filament extend laterally through a cut-out (96a) formed in a side surface of the insulation member (96) adjacent to the distal end thereof.

2. An electrosurgical instrument as claimed in claim 1, wherein the return electrode (98) is formed with a hood-like extension (98a) which extends over the surface of the insulation member (96) which is opposite the cut-out (96a).

3. An electrosurgical instrument as claimed in claim 1 or claim 2, wherein the single coiled filament is connected to a central conductor, the insulation member surrounding the central conductor.

4. Electrosurgical apparatus comprising a radio frequency generator and an electrosurgical instrument as claimed in any one of claims 1 to 3.

5. Apparatus as claimed in claim 4, wherein the radio frequency generator includes control means for varying the output power delivered to the electrodes.

6. Apparatus as claimed in claim 5, wherein the control means is such as to provide output power in first and second output ranges, the first output range being for powering the electrosurgical instrument for tissue desiccation, and the second output range being for powering the electrosurgical instrument for tissue removal by vaporisation.

7. Apparatus as claimed in claim 6, wherein the first output range is from about 150 volts to 200 volts, and the second output range is from about 250 volts to 600 volts, the voltage being peak voltages.

## Patentansprüche

1. Elektrochirurgisches Instrument für die Bearbeitung von Gewebe in Anwesenheit eines elektrisch leitfähigen, fluiden Mediums, wobei das Instrument einen Instrumentenschaft (10) und eine Elektrodenbaugruppe (E10) an einem Ende des Schaftes umfasst, wobei die Elektrodenbaugruppe eine Gewebebearbeitungselektrode (94) und eine Rückführelektrode (98) umfasst, die mittels eines Isolationselementes (96) elektrisch von der Gewebebearbeitungselektrode isoliert ist, die Gewebebearbeitungselektrode liegt an dem distalen Endabschnitt des Instrumentes frei, wobei das freiliegende Ende der Gewebebearbeitungselektrode durch ein einzelnes gewickeltes Filament (94) gebildet wird und die Rückführelektrode eine Fluid-Kontaktoberfläche besitzt, die proximal von dem freiliegenden Ende der Gewebebearbeitungselektrode durch das Isolationselement beabstandet ist, **dadurch gekennzeichnet, dass** sich die Wicklungen des einzelnen gewickelten Filamentes seitlich durch einen Freischnitt (96a) erstrecken, der in einer Seitenfläche des Isolationselementes (96) und benachbart zu dessen distalen Ende gebildet ist.

2. Elektrochirurgisches Instrument nach Anspruch 1, wobei die Rückführelektrode (98) mit einer hakenartigen Verlängerung (98a) ausgebildet ist, die sich über die Oberfläche des Isolationselementes (96) erstreckt, das dem Freischnitt (96a) gegenüberliegt.

3. Elektrochirurgisches Instrument nach Anspruch 1 oder 2, wobei das einzelne gewickelte Filament mit einem Mittenleiter verbunden ist und das Isolationselement den Mittenleiter umgibt.

4. Elektrochirurgische Vorrichtung umfassend einen Radiofrequenzgenerator und ein elektrochirurgisches Instrument nach einem der Ansprüche 1 bis 3.

5. Vorrichtung nach Anspruch 4, wobei der Radiofrequenzgenerator ein Steuerungsmittel zum Verändern der an die Elektroden gelieferten Ausgangsleistung umfasst.

6. Vorrichtung nach Anspruch 5, wobei das Steuerungsmittel ausgebildet ist, um eine Ausgangsleistung in einem ersten und einem zweiten Ausgangsbereich zu liefern, der erste Ausgangsbereich zum Versorgen des elektrochirurgischen Instrumentes für eine Gewebeentwässerung dient und der zweite Ausgangsbereich zum Versorgen des elektrochirurgischen Instrumentes für eine Gewebeentfernung durch Verdampfung dient.

7. Vorrichtung nach Anspruch 6, wobei der erste Ausgangsbereich zwischen 150 Volt bis 200 Volt und der zweite Ausgangsbereich zwischen 250 Volt bis 600 Volt liegt und wobei die Spannungen Spitzenspannungen sind.

## Revendications

1. Un instrument électrochirurgical pour le traitement de tissu en présence d'un milieu fluide électriquement conducteur, l'instrument comprenant un arbre d'instrument (10) et un ensemble d'électrodes (E10) à une extrémité de l'arbre, l'ensemble d'électrodes comprenant une électrode de traitement de tissu (94) et une électrode de référence (98) qui est isolée électriquement de l'électrode de traitement de tissu au moyen d'un élément isolant (96), l'électrode de traitement de tissu étant exposée à la partie d'extrémité distale de l'instrument, l'extrémité exposée de l'électrode de traitement de tissu étant constituée par un filament à simple boudinage (94) et l'électrode de référence ayant une surface de mise en contact de fluide, espacée de manière proximale de l'extrémité exposée de l'électrode de traitement de tissu par l'élément isolant, **caractérisé en ce que** les boudinages du filament à simple boudinage s'étendent latéralement à travers une découpe (96a) formée dans une surface latérale de l'élément isolant (96) adjacent à son extrémité distale.

2. Instrument électrochirurgical selon la revendication 1, dans lequel l'électrode de référence (98) est formée avec un prolongement de type capot (98a) qui s'étend sur la surface de l'élément isolant (76) qui est opposée à la découpe (96a).

3. Instrument électrochirurgical selon la revendication 1 ou 2, dans lequel le filament à simple boudinage est connecté à un conducteur central, l'élément isolant entourant le conducteur central.

4. Appareil électrochirurgical comprenant un générateur de radiofréquence et un instrument électrochirurgical tel que revendiqué dans l'une quelconque des revendications 1 à 3.

5. Appareil selon la revendication 4, dans lequel le générateur de radiofréquence comprend des moyens de commande destinés à faire varier la puissance de sortie délivrée aux électrodes.

6. Appareil selon la revendication 5, dans lequel les moyens de commande sont tels qu'ils fournissent une puissance de sortie dans les première et seconde gammes de sortie, la première gamme de sortie étant destinée à alimenter l'instrument électrochirurgical pour la dessiccation de tissu, et la seconde gamme de sortie étant destinée à alimenter l'instrument électrochirurgical pour le retrait de tissu par vaporisation.

7. Appareil selon la revendication 6, dans lequel la première gamme de sortie est d'environ 150 volts à 200 volts, et la seconde gamme de sortie est d'environ 250 volts à 600 volts, la tension étant des tensions de crête.
